(19) Europäisches Patentamt
European Patent Office
Office européen des brevets

(11) Veröffentlichungsnummer : **0 150 435**
**B1**

(12) # EUROPÄISCHE PATENTSCHRIFT

(45) Veröffentlichungstag der Patentschrift : **02.03.88**

(51) Int. Cl.⁴ : **C 07 C118/02**

(21) Anmeldenummer : **84115708.4**

(22) Anmeldetag : **18.12.84**

(54) **Verfahren zur kontinuierlichen Herstellung von organischen Mono- und/oder Polyisocyanaten.**

(30) Priorität : **31.01.84 DE 3403204**

(43) Veröffentlichungstag der Anmeldung : **07.08.85 Patentblatt 85/32**

(45) Bekanntmachung des Hinweises auf die Patenterteilung : **02.03.88 Patentblatt 88/09**

(84) Benannte Vertragsstaaten : **BE DE FR GB IT NL**

(56) Entgegenhaltungen :
**EP-A- 0 065 727**
**DE-A- 2 624 285**
**FR-A- 1 112 607**
**FR-A- 2 353 521**
**US-A- 3 781 320**

(73) Patentinhaber : **BASF Aktiengesellschaft**
**Carl-Bosch-Strasse 38**
**D-6700 Ludwigshafen (DE)**

(72) Erfinder : **Ohlinger, Rainer, Dr.**
**Ziegelhaeusser Landstrasse 31**
**D-6900 Heidelberg (DE)**
Erfinder : **Schnez, Harald, Dr.**
**Sonnenblick 6**
**D-6430 Bad Hersfeld-Asbach (DE)**
Erfinder : **Pfannenstiehl, Ludwig**
**Liebfrauenstrasse 20**
**D-6719 Kirchheimbolanden (DE)**
Erfinder : **Blumenberg, Bernd, Dr.**
**Hermann-Muendler-Strasse 5**
**D-6710 Frankenthal (DE)**
Erfinder : **Raabe, Hans Joachim, Dr.**
**In der Dreispitz 8**
**D-6706 Wachenheim (DE)**

**EP 0 150 435 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Es ist bekannt, Isocyanate aus primären Aminen und Phosgen herzustellen. Die Umsetzung wird je nach Art der Amine entweder in der Gasphase oder in flüssiger Phase sowohl diskontinuierlich als auch kontinuierlich durchgeführt (W. Siefken, Liebigs Ann. 562, 75 (1949)).

Die bekannten Verfahren besitzen zahlreiche Nachteile. Werden niedere Temperaturen bei der anfänglichen Phosgenierung (Kaltphosgenierung) angewendet, so stellt die Freisetzung großer Mengen Phosgen während der Erhöhung der Temperatur auf die Endphosgenierungstemperatur (Heißphosgenierungstemperatur) ein schwierig zu handhabendes Problem dar, das zusätzlich durch die hohe Toxizität des Phosgens erschwert wird. Niedertemperaturverfahren sind auch mit einem anderen Nachteil behaftet, nämlich dem, daß die Reaktionsgeschwindigkeit verhältnismäßig gering ist, was zu großen Reaktionsvolumina führt. In den Zweistufenverfahren kann das Endprodukt und das in der ersten Stufe als Zwischenprodukt gebildete Carbamylchlorid mit etwas Ausgangsamin unter Bildung von substituierten Harnstoffen und Polyharnstoffen oder anderen unerwünschten Nebenprodukten reagieren.

Nach anderen kontinuierlichen Verfahren zur Herstellung von Isocyanaten wird ein Teil der Reaktionslösung bei erhöhtem Druck und erhöhter Temperatur im Kreislauf geführt. Nach Angaben der DE-AS 1 037 444 (US 2 822 373) wird hierbei die Phosgenlösung vor dem Eintritt in das Reaktionsgefäß mit einer Aminlösung vermischt. Hierbei muß sich am Vereinigungspunkt beider Lösungen eine bestimmte Turbulenz ausbilden. Dabei können jedoch im Strömungsrohr leicht Verstopfungen auftreten, die keine Turbulenz mehr gestatten.

Die DE-PS 1 175 666 (GB 827 376) beschreibt ein Verfahren zur kontinuierlichen Herstellung von Isocyanaten bei erhöhtem Druck in einem Reaktionsrohr, wobei die Ausgangsstoffe bei einem Druck von über 3 bar in das Reaktionsrohr eingeführt, bei ansteigender Temperatur zur Reaktion gebracht und die Reaktionsmischung zur Gewinnung der Isocyanate am Ende des Reaktionsrohres über ein Entspannungsventil abgezogen werden. Da das rückgeführte Phosgen keiner Chlorwasserstoff-Reinigung unterworfen wird, kommt es zur Salzbildung und Ablagerungen an der Mischdüse.

Zur Verbesserung dieses Verfahrens wird gemäß DE-AS 1 593 412 (GB 1 136 656 und GB 1 141 910) die Zersetzung des Carbamidsäurechlorids in Isocyanat und Chlorwasserstoff und die Entfernung des gesamtem Chlorwasserstoffs und des überschüssigen Phosgens aus dem Reaktionsgemisch in einer Destillationskolonne bei Temperaturen von 120 bis 180 °C und einem Druck von 10 bis 50 bar unter Rückfluß des Phosgens durchgeführt.

Eine Mischvorrichtung, bestehend aus einer Kammer und einem koaxial angeordneten, mit Schaufeln ausgestatteten Rotor, dessen Schaufelenden mit der inneren Kammerwand einen geringen Abstand bilden, zur Herstellung von organischen Isocyanaten nach einem Einstufenverfahren ist Gegenstand der DE-OS 19 56 777 (US 3 781 320 und US 3 887 167). Nach Angaben dieser Publikation ist eine Chlorwasserstoffabtrennung aus der Reaktionsmischung nicht erforderlich, da dessen Anwesenheit für das Verfahren nicht nachteilig ist.

Nach Angaben der DE-OS 21 12 181 (US 3 829 458) werden organische Isocyanate aus primären organischen Aminen und Phosgen in einem inerten organischen Lösungsmittel kontinuierlich in einem oder mehreren, Füllkörper enthaltenden Reaktionsgefäßen, vorzugsweise unter Rückführung der Reaktionsmischung, in der sogenannten Übergangsströmung hergestellt. Mit Hilfe des relativ einfachen Verfahrens gelingt es, Isocyanate in hoher Raum-Zeit-Ausbeute herzustellen. Aber auch bei diesem Verfahren bereitete das Einbringen der Amine in die phosgenhaltige Reaktionslösung gewisse Schwierigkeiten, da bei manchen Aminen eine optimale Durchmischung der Ausgangskomponenten nur schwer erreicht werden kann. Werden jedoch die Reaktionskomponenten nicht ausreichend durchmischt, so entstehen Aminhydrochloride und Harnstoffe als Nebenprodukte, die sich teilweise auf den Füllkörpern absetzen und zu Verstopfungen der Füllkörpersäulen führen können.

Eine Reihe der bekannten Herstellungsverfahren hat zwar beträchtliche technische Erfolge erzielt, doch besitzen alle diese Methoden den Nachteil, daß übermäßige Mengen an Polymeren und anderen unerwünschten Nebenprodukten gebildet werden. Durch die Bildung der Nebenprodukte kann die Isocyanatausbeute bzw. die Produktqualität erheblich vermindert werden.

Gute Ergebnisse werden auch nach dem in der DE-OS 26 24 285 (US 4 128 569) beschriebenen Verfahren erzielt. Hierbei wird das Phosgen der im Kreislauf geführten Reaktionslösung zugemischt und das erhaltene Reaktionsgemisch und die Amine oder Aminlösung der Misch- und Reaktionszone so zugeführt, daß in dieser eine Energiedissipationsdichte von 5 bis 1 000 kJ je $m^3$ rückgeführten Reaktionsgemisches plus zugeführter Aminlösung erzeugt wird. Aber auch nach diesem Verfahren kann die gewünschte Phosgenierungsqualität insbesondere im Hinblick auf die Nebenproduktbildung nicht erzielt werden.

Die Aufgabe der vorliegenden Erfindung bestand darin, Mischkreisverfahren zur kontinuierlichen Herstellung von Isocyanaten, insbesondere das Verfahren gemäß DE-OS 26 24 285, zu verbessern und vorhandene Mängel ganz oder zumindest teilweise zu beseitigen.

Diese Aufgabe konnte überraschenderweise durch eine Verminderung des Chlorwasserstoffgehalts und Erhöhung des Phosgengehalts der Reaktionsmischung gelöst werden.

Gegenstand der Erfindung ist somit ein Verfahren zur kontinuierlichen Herstellung von organischen Isocyanaten durch Umsetzung von organischen Aminen mit Phosgen in Gegenwart von organischen Lösungsmitteln unter Druck bei erhöhter Temperatur, wobei Chlorwasserstoff abgetrennt und die Reaktionsmischung teilweise im Kreislauf geführt wird, das dadurch gekennzeichnet ist, daß der Chlorwasserstoffgehalt der nach der Chlorwasserstoff-Abtrennung zur Aminzugabe rückgeführten Reaktionsmischung von der Aminzugabe gleich oder kleiner als 0,5 Gew-%, vorzugsweise 0,01 bis 0,4 Gew.-%, bezogen auf das Gesamtgewicht der Reaktionsmischung, ist und das Molverhältnis von Phosgen zu $NH_2$ -Gruppe der organischen Amine 12 bis 200 : 1 beträgt.

Durch das erfindungsgemäße Verfahren kann in vorteilhafter Weise die intermediäre Salzbildung vermieden, die Nebenproduktbildung im wesentlichen unterdrückt und dadurch die Isocyanatkonzentration im Reaktor erhöht werden. Hiermit verbunden ist eine Energieeinsparung bei der Aufarbeitung der erhaltenen Reaktionsmischung. Der Phosgenierungsreaktor kann im Vergleich zum Kaskadenverfahren verkleinert und damit die Raum-Zeit-Ausbeuten erhöht werden. Das entstehende Chlorwasserstoffgas wird durch Rektifizierung unter hohem Druck abgetrennt.

Das erfindungsgemäße Verfahren ist ganz allgemein auf die Herstellung von organischen Isocyanaten, die durch Umsetzung von Aminen mit Phosgen erhalten werden können, anwendbar. So können beispielsweise aliphatische, cycloaliphatische, aliphatisch-aromatische, aromatische, Mono-, Di- und/oder Polyisocyanate aus den entsprechenden organischen Mono-, Di- und/oder Polyaminen hergestellt werden.

Geeignete organische Monoaminoverbindungen besitzen die Formel $R{-}NH_2$, wobei R einen gegebenenfalls substituierten, einwertigen aliphatischen, cycloaliphatischen oder vorzugsweise aromatischen Rest mit 1 bis 20, vorzugsweise 6 bis 12 Kohlenstoffatomen bedeutet. Genannt seien beispielsweise aliphatische Monoamine, wie Methylamin, Ethylamin, Butylamin, Octylamin und Stearylamin, cycloaliphatische Monoamine, wie Cyclohexylamin und insbesondere aromatische Monoamine, wie Anilin, Toluidine, Naphthylamine, Chloraniline und Anisidine.

Vorzugsweise werden jedoch die zur Herstellung von Polyurethanen technisch bedeutsamen Di- und Polyisocyanate aus den entsprechenden Di- und Polyaminen nach dem neuen Verfahren hergestellt. Geeignete Diaminoverbindungen besitzen die Formel $H_2N{-}R'{-}NH_2$, wobei R' bedeutet einen zweiwertigen aliphatischen oder cycloaliphatischen Rest mit 2 bis 18, insbesondere 4 bis 13 Kohlenstoffatomen oder vorzugsweise einen zweiwertigen aromatischen Rest, der aus einem oder mehreren aromatischen Kernen, die 6 bis 18 Kohlenstoffatome besitzen, besteht, die direckt miteinander verknüft sind, oder gegebenenfalls über zweiwertige Brückenglieder, wie —O—,

$$-SO_2-,\ -CH_2-\ \text{und}\ -CH_3-\overset{|}{\underset{|}{C}}-CH_3$$

miteinander verbunden sind. Die Diamino- und/oder Polyaminoverbindungen können einzeln oder als Mischungen verwendet werden.

Solche aliphatische, cycloaliphatische oder insbesondere aromatische Diaminoverbindungen sind beispielsweise : 1,4-Diaminobutan, 1,6-Diaminohexan, 1,10-Diamino-decan, 1,12-Diamino-dodecan, 1,4- bzw. 1,3-Diaminocyclohexan, 4,4'-Diamino-dicyclohexyl, 4,4'-, 2,4'-, 2,2'-Diamino-dicyclohexylmethan, 3,3'-Dimethyl- 4,4'-diaminodicyclohexylmethan, 4,4'-Diamino-diphenyl, 1,4- bzw. 1,3-Phenylendiamin, 1,5- bzw. 1,8-Naphthylendiamin, 2,4- bzw. 2,6-Toluylendiamin und 2,2'-, 2,4- bzw. 4,4'-Diamino-diphenylmethan.

Als Polyamine kommen beispielsweise in Betracht Tri (p-aminophenyl)-methan, 2,4,6-Triamino-toluol und Kondensationsprodukte, die aus gegebenenfalls substituierten Anilinderivaten und Aldehyden bzw. Ketonen in Gegenwart von Säuren erhalten werden, wie Polyphenyl-polymethylen-polyamine.

Als organische Amine werden vorzugsweise verwendet 1,6-Hexamethylen-diamin, Mischungen aus 1,6-Hexamethylen-, 2-Methyl-1,5-pentamethylen- und 2-Ethyl-1,4-butylen-diamin, 3-Aminomethyl-3,5,5-trimethylcyclohexylamin, 2,4'-, 4,4', 2,2'-Diamino-diphenylmethan oder Mischungen aus mindestens zwei der genannten Isomeren, 2,4- und 2,6-Toluylendiamin oder deren Gemische, Polyphenyl-polymethylen-polyamine oder Mischungen aus Diamino-diphenylmethanen und Polyphenyl-polymethylen-polyaminen.

Das erfindungsgemäße Verfahren eignet sich besonders gut zur Herstellung von aromatischen Di- und/oder Polyisocyanaten aus den entsprechenden Aminen und wird daher hierzu auch vorzugsweise angewandt.

Als andere Ausgangskomponente wird gasförmiges und/oder vorzugsweise flüssiges Phosgen verwendet. Das flüssige Phosgen kann als solches oder in Verdünnung mit zur Phosgenierung geeigneten Lösungsmitteln, wie z. B. Monochlor-, Dichlorbenzol, Xylol, Toluol u. a. umgesetzt werden. Das Molverhältnis von Amin zu Phosgen wird zweckmäßigerweise so bemessen, daß pro $NH_2$-Gruppe 12 bis 200 Mol, vorzugsweise 12 bis 98 Mol und insbesondere 40 bis 80 Mol, Phosgen in der Reaktionsmischung vorliegen.

Als inerte organische Lösungsmittel kommen Verbindungen in Betracht, in welchen die Amine und das Phosgen mindestens teilweise löslich sind.

Besonders bewährt haben sich chlorierte, aromatische Kohlenwasserstoffe, wie z. B. Chlorbenzol, o-Dichlorbenzol, p-Dichlorbenzol, Trichlorbenzole, die entsprechenden Chlortoluole und -xylole, Chlorethylbenzol, Monochlordiphenyl, α- bzw. β-Naphthylchlorid, Benzoesäurealkylester, Phthalsäuredialkylester, wie iso-Diethylphthalat, Toluol und Xylole. Die Lösungsmittel können einzeln oder als Gemische verwendet werden. Zweckmäßig wird ein Lösungsmittel verwendet, das einen niedrigeren Siedepunkt besitzt als das herzustellende Isocyanat, damit das Lösungsmittel leicht durch Destillation vom Isocyanat abgetrennt werden kann. Die Menge an Lösungsmittel wird vorteilhafterweise so bemessen, daß die Reaktionsmischung einen Isocyanatgehalt von 4 bis 40 Gew.-%, vorzugsweise von 20 bis 30 Gew.-%, bezogen auf das Gesamtgewicht der Reaktionsmischung, aufweist.

Die Amine können als solche oder vorzugsweise gelöst in organischen Lösungsmitteln zur Anwendung kommen. Insbesondere verwendet man jedoch Aminlösungen mit einem Amingehalt von 5 bis 50 Gew.-%, vorzugsweise von 15 bis 35 Gew.-%, bezogen auf das Gesamtgewicht der Aminlösung.

Die Umsetzung führt man zweckmäßigerweise bei Temperaturen von 100 bis 220 °C, vorzugsweise von 120 bis 180 °C und in einen Druckbereich von 5 bis 100 bar, vorzugsweise 15 bis 50 bar durch. Die bei dem erfindungsgemäßen Verfahren angewendete Temperatur liegt über der Zersetzungstemperatur des als Zwischenprodukt durch die Reaktion des Phosgens mit Amin gebildeten Carbamylchlorids. Einer Erhöhung des Drucks sind nur technische und gegebenenfalls sicherheitstechnische Grenzen gesetzt, wobei mit höherem Druck keine Ausbeutesteigerung mehr erzielt wird. Die Rektifikation des abgetrennten Chlorwasserstoffs wird bei hohem Druck jedoch wesentlich erleichtert.

Bei der Durchführung des erfindungsgemäßen Verfahrens wird die Reaktionsmischung, die aus Lösungsmittel, gelöstem Isocyanat, Phosgen, Chlorwasserstoff sowie Nebenprodukten der Phosgenierung besteht, im Kreislauf, vorzugsweise in einem Druckmischkreis, mittels einer Umwälzpumpe oder vorzugsweise aufgrund der Dichteunterschiede durch Naturumlauf geführt, wobei die rückgeführte Reaktionsmischung teilweise über eine Pumpe und Düse in die Misch- und Reaktionszone eingeleitet wird. Über den Kreislaufstrom wird der Reaktionsmischung frisches Phosgen und gegebenenfalls lösungsmittelhaltiges Phosgen zugemischt, wobei nach einer vorteilhaften Ausgestaltung des Verfahrens das frische Phosgen, vorzugsweise in flüssiger Form, zusammen mit dem rückgeführten und gereinigten Phosgen aus der Chlorwasserstoffabtrennung in den über die Düse führenden Teilstrom der Reaktionsmischung einverleibt wird. Das Amin oder vorzugsweise die Aminlösung wird über die Düse in die Reaktionsmischung eingeleitet. Ein der gesamten Flüssigkeitsbeschickung entsprechender Volumenstrom an Reaktionsmischung wird dem Kreislauf als Produktlösung zur weiteren Aufarbeitung und Isolierung des Isocyanats entnommen. Zur Abtrennung des bei der Phosgenierung frei werdenden Chlorwasserstoffs ist ein Entgasungsraum im Kreislauf erforderlich, mit dessen Hilfe der Chlorwasserstoffgehalt der Reaktionsmischung von der Aminzugabe auf weniger als 0,5 Gew.-%, vorzugsweise 0,01 bis 0,4 Gew.-% und insbesondere 0,1 bis 0,3 Gew.-%, bezogen auf das Gesamtgewicht der Reaktionsmischung, erniedrigt wird.

Wird die Reaktionsmischung nach dem Prinzip des Naturumlaufs gefördet, so ist es zweckmäßig, wenn der zylinderförmig ausgebildete Apparateteil zwischen Düse und Entgasungsraum, in dem der Kreislaufstrom senkrecht aufsteigt, eine Länge von 5 bis 25 m, vorzugsweise 10 bis 15 m und einen davon nahezu unabhängigen Innendurchmesser von 5 bis 150 cm, vorzugsweise 10 bis 100 cm, besitzt. Auf die in der Abbildung gezeichnete Umwälzpumpe 18 kann dann verzichtet werden.

Die kontinuierlich von Chlorwasserstoff gereinigte Reaktionsmischung enthält somit zweckmäßigerweise pro 100 Gew.-Teile Reaktionsmischung 9,5 bis 86, vorzugsweise 40 bis 50 Gew.-Teile Lösungsmittel, 4 bis 40, vorzugsweise 20 bis 30 Gew.-Teile organisches Isocyanat, 10 bis 50, vorzugsweise 20 bis 40 Gew.-Teile Phosgen und 0,01 bis 0,5, vorzugsweise 0,01 bis 0,4 Gew.-Teile Chlorwasserstoff.

Das Volumen des Reaktionskreislaufs wird in seinen Größenabmessungen so ausgelegt, daß mittlere Verweilzeiten von etwa einer Minute bis einer Stunde, vorzugsweise 5 Minuten bis 0,5 Stunden, bezogen auf den Volumenstrom der ausgetragenen Produktlösung, eingestellt werden können.

Die Reaktionsmischung wird in einer solchen Menge im Kreislauf gehalten, daß das Volumenverhältnis der Gesamtmenge aus der im Kreislauf geführten Reaktionsmischung plus zugesetztem Phosgen plus gegebenenfalls zugesetztem lösungsmittelhaltigem Phosgen zur Menge an zugeführter Aminlösung 300 : 1 bis 1 : 1, vorzugsweise 100 : 1 bis 5 : 1 beträgt.

Der Anteil des aus im Kreislauf geführten Reaktionsmischung, zugeführtem Phosgen und gegebenenfalls zugegebenem lösungsmittelhaltigem Phosgen bestehenden Reaktionsgemisches, der über die Düse geführt wird, kann zwischen 5 und 100 Gew.-%, vorzugsweise 10 und 50 Gew.-%, des gesamten in den Reaktionskreislauf gegebenen Reaktionsgemisches ausmachen. Im Extremfall kann das gesamte Reaktionsgemisch über die Düse gefahren werden. Der durch die Düse geleitete Anteil des Reaktionsgemisches wird stark beschleunigt, so daß er mit hoher Relativgeschwindigkeit zum Inhalt des Reaktionsrohres als Treibstrahl aus der Düse austritt. Durch Zuführen bis an die Düsenaustrittsstelle wird die Aminlösung mit der Reaktionsmischung in äußerst kurzer Zeit intensiv vermischt.

Um optimale Reaktionsgeschwindigkeiten und Isocyanatausbeuten zu erhalten, wird zweckmäßigerweise in der Misch- und Reaktionszone ein Energiedissipationsdichte von 5 bis 1 000, vorzugsweise von 50 bis 400 kJ je $m^3$ rückgeführter Reaktionsmischung plus zugeführter Aminlösung eingestellt. Diese Energiedissipationsdichte wird erzeugt, wenn man den durch die Düse geführten Anteil der Reaktionsmischung mit einer Düsenaustrittsgeschwindigkeit von 1 bis 60 m/sek, vorzugsweise 20 bis 40 m/sek, und das Amin bzw. die Aminlösung mit einer Austrittsgeschwindigkeit von 0,3 bis 30 m/sek, vorzugsweise

5 bis 20 m/sek durch das Aminzuleitungsrohr der Misch- und Reaktionszone zuführt. Die Misch- und Reaktionszone ist gekennzeichnet durch den beschleunigten Massenstrom von rückgeführter Reaktionsmischung und zugeführter Aminlösung sowie dem darin eingemischten Massenstrom des Kreislaufs, wobei die vereinigten Flüssigkeitsströme die oben angegebenen Energiedissipationsdichten aufweisen. Die Misch- und Reaktionszone hat einen mittleren Durchmesser, der dem 3- bis 30-fachen, vorzugsweise 10- bis 25-fachen des mittleren Durchmessers des Treibstrahles der Reaktionsmischung entpricht. Unter mittlerem Durchmesser des Treibstrahles ist der Durchmesser eines flächengleichen Kreises zu verstehen, der aus den Querschnittflächen der Düsenöffnungen, beispielsweise von Ring- oder Schlitzdüsen, der Mischzone ermittelt wird. Die Misch- und Reaktionszone kann einen konstanten oder sich in der Strömungsrichtung veränderten Querschnitt haben. Die Misch- und Reaktionszone kann in verschiedenen Formen gestaltet werden, wobei diese Form zweckmäßigerweise der verwendeten Düsenform angepaßt wird. Im allgemeinen verwendet man Kegelsegmente oder vorzugsweise zylindrische Rohre, sofern die Misch- und Reaktionszone als zylindrisches Rohr ausgestaltet ist, soll ihre Länge das 1- bis 20-fache, vorzugsweise das 1,5- bis 5-fache ihres Durchmessers betragen. Sofern die Misch- und Reaktionszone keinen kreisförmigen oder über ihre Länge keinen konstanten Querschnitt aufweist, soll ihre Länge das 1- bis 20-fache, vorzugsweise das 1,5- bis 5-fache des hydraulischen Durchmessers betragen. Unter hydraulischem Durchmesser ist der Durchmesser eines zylindrischen Rohres zu verstehen, das bei gleichen durchgesetzten Mengen und gleicher Länge den gleichen Druckverlust zeigt wie die betreffende Misch- und Reaktionszone.

Die Misch- und Reaktionszone ist Teil des Reaktionskreislaufvolumens, dessen Größen durch die vorstehend genannten mittleren Verweilzeiten gekennzeichnet ist. Die Misch- und Reaktionszone muß nicht notwendigerweise als ein separates Reaktionsgefäß ausgeführt werden, sondern kann z. B. auch als Reaktionsrohr ausgebildet und Teil des Kreislauf-Leitungssystems sein.

Der Entgasungsraum zum Entweichen des bei der Phosgenierung entstehenden Chlorwasserstoffs ist zweckmäßigerweise über dem Reaktionsraum angeordnet. Wird als Reaktionsraum ein separates Reaktionsgefäß verwendet, so bietet es sich an, als Entgasungsraum den Oberteil dieses Gefäßes zu verwenden und das Phosgen und Lösungsmitteldämpfe enthaltende Chlorwasserstoffgas an dieser Stelle abzuziehen. Statt dessen kann jedoch auch ein separates Entgasungsgefäß im Kreislaufsystem dem Reaktionsraum nachgeschaltet sein.

Eine bevorzugte Ausführungsform des erfindungsgemäßen Herstellungsverfahrens wird anhand der Figur des Verfahrensschemas nochmal näher erläutert :

In der Figur bedeuten

1 Zuleitung für Amin
2 Zuleitung für Lösungsmittel
3 Zuleitung für aminhaltiges Lösungsmittel
4 Kreislaufleitung für Reaktionsmischung
5 Kreislaufleitung für über die (Treibstrahl) düse rückgeführte Reaktionsmischung
5' Kreislaufleitung für rückgeführte Reaktionsmischung
6 Ableitung für Chlorwasserstoff, Phosgen und Lösungsmitteldämpfe
7 Ableitung für Chlorwasserstoff
8 Rückleitung für Phosgen und kondensiertes Lösungsmittel
9 Ableitung von Produktlösung zur Isolierung des Isocyanates
10 Zuleitung für Phosgen
11 Mischbehälter
12 Dosierpumpe
13 Düse (Treibstrahldüse)
14 Misch- und Reaktionszone
15 Entgasungsraum
16 Rektifikationskolonne
17, 18 } Umwälzpumpen
19 Wärmeaustauscher

Durch die Kreislaufleitungen 4, 5 und 5', die mindestens mit soviel Reaktionsmischung gefüllt sind, daß die Treibstrahldüse 13 vollständig in die Reaktionsmischung eintaucht, wird über die Umwälzpumpe 17, den Wärmeaustauscher 19 und die Treibstrahldüse 13 sowie gegebenenfalls die Umwalzpumpe 18 Reaktionsmischung im Kreislauf geführt, wobei gegebenenfalls lösungsmittelhaltiges Phosgen durch die Rückleitung 8 und frisches Phosgen über die Zuleitung 10 und Rückleitung 8 der Reaktionsmischung zugemischt wird. Die Reaktionsmischung der Kreislaufleitung 5 wird geteilt in Teilströme 5' und 5. Der Teilstrom 5 wird direkt über die Kreislaufleitung 5, die Umwälzpumpe 17, dem Wärmeaustauscher 19, angereichert mit frischem und rückgeführtem Phosgen der Düse 13 zugeführt und stark beschleunigt in Form eines Treibstrahls in die Reaktionsmischung eingedüst, während dem über die Kreisleitung 5' geführten anderen Teilstrom über die Ableitung 9 isocyanathaltige Reaktionsmischung entnommen wird. Durch die Zuleitung 1 wird Amin und durch die Zuleitung 2 Lösungsmittel in den Mischbehälter 11 gefüllt.

Die Aminlösung wird über die Dosierpumpe 12 durch die Zuleitung 3 eingebracht und mit der Reaktionsmischung in der Misch- und Reaktionszone 14 intensiv gemischt und zur Reaktion gebracht. Durch die Ableitung 6 entweichen aus dem Entgasungsraum 15 die flüchtigen Bestandteile, nämlich Chlorwasserstoff, teilweise überschüssiges Phosgen und Lösungsmitteldampf, in eine Rektifikationskolonne 16. Das Lösungsmittel und Phosgen wird dort gereinigt und durch die Rückleitung 8 in die Reaktionsmischung eingeleitet, während der gasförmige Chlorwasserstoff über die Ableitung 7 abgeführt wird. Die nach dem erfindungsgemäßen Verfahren hergestellte isocyanathaltige Reaktionsmischung wird durch die Ableitung von Produktlösung 9 abgezogen und mit Hilfe von üblichen Reinigungsmethoden zu Isocyanaten aufgearbeitet.

Das erfindungsgemäße Verfahren wird durch die folgenden Beispiele erläutert.

Beispiele 1 bis 5

Die Verfahrensanordnung ist aus der Figur zu ersehen. As Druckmischkreis 4, 5, 5' wurde ein Reaktionsrohr 100 mm Durchmesser und einem Volumen von 160 l verwendet. Der Entgasungsraum bestand aus einem Behälter mit einem Volumen von 1 000 l, der zu 5 bis 60 Vol.-% mit Reaktionsmischung gefüllt war. Die Reaktionsmischung wurde über die Kreislaufleitungen 4, 5 und 5' nach dem Naturumlaufprinzip gefördert. Die Umwälzpumpe 18 erübrigte sich daher. Das flüssig zugeführte Frischphosgen wurde mit rückgeführtem Phosgen gemischt über die Rückleitung 8 der Reaktionsmischung zwischen Umwälzpumpe 17 und Wärmeaustauscher 19 einverleibt. Die Reaktionsmischung der Kreislaufleitung 5 wurde mit einer Kreiselpumpe 17 beschleunigt und durch eine Treibstrahldüse, die einen Innendurchmesser von 6 bis 16 mm aufwies und in die Reaktionsmischung eintauchte, eingedüst. Die Aminlösung wird durch ein Zuleitungsrohr von 2 bis 4 mm Durchmesser dem Treibstrahl einverleibt. Zu Beginn der Umsetzung wird der Druckmischkreis mit Phosgen gesättigtem Monochlorbenzol beschickt.

Die zur Herstellung der Isocyanate verwendeten Amine, die Durchflußmengen, Reaktionsbedingungen und Versuchsergebnisse sind in der folgenden Tabelle zusammengefaßt.

(Siehe Tabelle Seite 7 ff.)

Tabelle

| Beispiel | 1 | 2 | 3 | 4 | 5 |
|---|---|---|---|---|---|
| Durchmesser der Treibstrahldüse [mm] | 10 | 10 | 10 | 10 | 10 |
| Durchmesser der Aminzuleitung [mm] | 4 | 4,5 | 3,5 | 4 | 4 |
| Flüssigkeitsvolumen des Druckmischkreises [l] | 500 | 600 | 700 | 500 | 800 |
| Amin | Roh-MDA | Roh-MDA | DIPPA | TDA | HMDA |
| Amingehalt der Basenlösung [Gew.-%] | 30 | 25 | 34 | 22 | 10 |
| Temperatur der Basenlösung [°C] | 170 | 127 | 136 | 170 | 185 |
| Zufuhr an Basenlösung 3 [kg/h] | 500 | 295 | 542 | 460 | 400 |
| Zufuhr an Phosgen 10 [kg/h] | 150 | 76 | 100 | 114 | 70 |
| Umlaufmenge an Reaktionsmischung insgesamt [$m^3$/h] | 30 | 36 | 24 | 28 | 22 |
| Durchlaufmenge durch Treibstrahldüse 13 [$m^3$/h] | 8 | 3,5 | 4,5 | 6 | 7 |
| Lineargeschwindigkeit des Treibstrahls [m/s] | 28 | 12 | 16 | 21 | 24,8 |
| Geschwindigkeit der Aminzufuhr [m/s] | 11 | 5 | 15 | 10 | 8,9 |
| Temperatur im Druckmischkreis [°C] | 130 | 123 | 120 | 130 | 180 |
| Druck im Druckmischkreis [bar] | 14,5 | 11 | 12 | 14,5 | 24,8 |
| Zusammensetzung der Reaktionsmischung nach Einmündung der Rückleitung 8 pro 100 Gew.-Teile | | | | | |
| Monochlorbenzol [Gew.-Teile] | 41,4 | 52,4 | 32 | 41,5 | 60 |
| Isocyanat " | 19,5 | 12,3 | 29,7 | 19 | 5 |
| Phosgen " | 38,6 | 35,0 | 38 | 39 | 35 |
| Chlorwasserstoff " | 0,4 | 0,25 | 0,3 | 0,45 | 0,1 |
| Druck in der Zuleitung 5 nach dem Wärmeaustauscher und vor der Treibstrahldüse [bar] | 20,5 | 15,5 | 16,5 | 19 | 29,4 |

In der Tabelle bedeuten :
Roh-MDA : Mischung aus Diphenylmethan-diaminen und Polyphenyl-polymethylen-Polyaminen
DIPPA : 2,6-Diisopropyl-phenylamin-1
TDA : Mischung aus 2,4- und 2,6-Toluylen-diamin im Gewichtsverhältnis 80 : 20
HMDA : Hexamethylen-diamin-1,6

Tabelle (Fortsetzung)

| Beispiel | 1 | 2 | 3 | 4 | 5 |
|---|---|---|---|---|---|
| Lösungsmittel-Phosgen-Rückführung 8 aus der Rektifikationskolonne 16: 85 %iges $COCl_2$ [kg/h] | 610 | 380 | 620 | 560 | 650 |
| Laufzeit [h] | 1400 | 48 | 170 | 120 | 72 |
| Ausbeute [%] | 100 | 100 | 99,5 | 96 | 92 |
| Isocyanat | Roh-MDI | Roh-MDI | DIPPI | TDI | HMDI |

In der Tabelle bedeuten :

Roh-MDI : Mischung aus Diphenylmethan-diisocyanaten und Polyphenyl-polymethylen-polyisocyanaten mit einem NCO-Gehalt von 31 Gew.%

DIPPI : 2,6-Diisopropyl-phenylisocyanat-1

TDI : Mischung aus 2,4- und 2,6-Toluylen-diisocyanat im Gewichtsverhältnis 80 : 20

HMDI : Hexamethylen-diisocyanat-1,6

**Patentansprüche**

1. Verfahren zur kontinuierlichen Herstellung von organischen Isocyanaten durch Umsetzung von organischen Aminen mit Phosgen in Gegenwart von organischen Lösungsmitteln unter Druck bei erhöhter Temperatur, wobei Chlorwasserstoff abgetrennt und die Reaktionsmischung teilweise im Kreislauf geführt wird, dadurch gekennzeichnet, daß der Chlorwasserstoffgehalt der nach der Chlorwasserstoff-Abtrennung zur Aminzugabe rückgeführten Reaktionsmischung gleich oder kleiner als 0,5 Gew.-%, bezogen auf das Gesamtgewicht der Reaktionsmischung, ist und das Molverhältnis von Phosgen zu $NH_2$-Gruppe der organischen Amine 12 bis 200 : 1 beträgt.

2. Verfahren nach Anspruch 1, dadurch gekennzeichnet, daß der Chlorwasserstoffgehalt der Reaktionsmischung von 0,01 bis 0,4 Gew.-%, bezogen auf das Gesamtgewicht der Reaktionsmischung, beträgt.

3. Verfahren nach Anspruch 1, dadurch gekennzeichnet, daß die Umsetzung in einem Druckmischkreis durchgeführt wird.

4. Verfahren nach Anspruch 1, dadurch gekennzeichnet, daß die Reaktionsmischung aufgrund der Dichteunterschiede im Kreislauf geführt wird.

5. Verfahren nach Anspruch 1, dadurch gekennzeichnet, daß die Reaktionstemperatur 100 bis 220 °C beträgt.

6. Verfahren nach Anspruch 1, dadurch gekennzeichnet, daß der Druck 5 bis 100 bar beträgt.

7. Verfahren nach Anspruch 1, dadurch gekennzeichnet, daß 100 Gew.-Teile der rückgeführten Reaktionsmischung enthalten :

9,5 bis 86 Gew.-Teile organisches Lösungsmittel,
4 bis 40 Gew.-Teile organisches Isocyanat,
10 bis 50 Gew.-Teile Phosgen und
0,01 bis 0,5 Gew.-Teile Chlorwasserstoff.

8. Verfahren nach Anspruch 1, dadurch gekennzeichnet, daß das Volumenverhältnis der Gesamtmenge aus zugesetztem Phosgen, zugesetztem lösungsmittelhaltigem Phosgen und der im Kreislauf geführten Reaktionsmischung zu zugeführtem Amin oder Aminlösung 300 bis 1 : 1 beträgt.

9. Verfahren nach Anspruch 1, dadurch gekennzeichnet, daß man als organische Amine aliphatische, cycloaliphatische, aliphatische-aromatische, aromatische Mono-, Di- und/oder Polyamine verwendet.

10. Verfahren nach Anspruch 1, dadurch gekennzeichnet, daß man als organische Amine 1,6-Hexamethylen-diamin, Mischungen aus 1,6-Hexamethylen-, 2-Methyl-1,5-pentamethylen- und 2-Ethyl-1,4-butylendiamin, 3-Aminomethyl-3,5,5-trimethylcyclohexylamin, 2,4'-, 4,4'-, 2,2'-Diamino-diphenylmethan oder Mischungen aus mindestens zwei der genannten Isomeren, 2,4- und 2,6-Toluylendiamin oder deren Gemische, Polyphenyl-polymethylen-polyamine oder Mischungen aus Diamino-diphenylmethanen und Polyphenyl-polymethylen-polyaminen verwendet.

**Claims**

1. A process for the continuous preparation of an organic isocyanate by reacting an organic amine with phosgene in the presence of an organic solvent under superatmospheric pressure and at elevated temperatures, hydrogen chloride being separated off and some of the reaction mixture being circulated, wherein the hydrogen chloride content of the reaction mixture recycled, after removal of the hydrogen chloride, for the addition of amine is equal to or less than 0.5 % by weight, based on the total weight of the reaction mixture, and the molar ratio of phosgene to $NH_2$ groups of the organic amine is from 12 : 1 to 200 : 1.

2. A process as claimed in claim 1, wherein the hydrogen chloride content of the reaction mixture is from 0.01 to 0.4 % by weight, based on the total weight of the reaction mixture.

3. A process as claimed in claim 1, wherein the reaction is carried out in a forced mixing circulation.

4. A process as claimed in claim 1, wherein the reaction mixture is circulated on the basis of the density differences.

5. A process as claimed in claim 1, wherein the reaction temperature is from 100 to 220 °C.

6. A process as claimed in claim 1, wherein the pressure is from 5 to 100 bar.

7. A process as claimed in claim 1, wherein 100 parts by weight of the recycled reaction mixture contain : from 9.5 to 86 parts by weight of organic solvent, from 4 to 40 parts by weight of organic isocyanate, from 10 to 50 parts by weight of phosgene and from 0.01 to 0.5 parts by weight of hydrogen chloride.

8. A process as claimed in claim 1, wherein the volume ratio of the total amount of added phosgene, added solvent-containing phosgene and the circulated reaction mixture to added amine or amine solution is from 300 : 1 to 1 : 1.

9. A process as claimed in claim 1, wherein the organic amines used are aliphatic, cycloaliphatic, aliphatic-aromatic or aromatic mono-, di- and/or polyamines.

10. A process as claimed in claim 1, wherein the organic amine used is 1,6-hexamethylenediamine, a mixture of 1,6-hexamethylene-, 2-methyl-1,5-pentamethylene- and 2-ethyl-1,4-butylenediamine, 3-aminomethyl-3,5,5-trimethylcyclohexylamine, 2,4'-, 4,4'- or 2,2'-diaminodiphenylmethane or a mixture of two or more of the stated isomers, 2,4- or 2,6-toluylenediamine or a mixture of these, a polyphenylpolymethylenepolyamine or a mixture of diaminodiphenylmethanes and polyphenylpolymethylenepolyamines.

**Revendications**

1. Procédé pour la préparation en continu d'isocyanates organiques par la réaction d'amines organiques et du phosgène sous pression et à température accrue, en présence de solvants organiques, dans lequel le mélange réactionnel circule en partie en circuit fermé et de l'hydrogène chloré est séparé, caractérisé en ce que la teneur en hydrogène chloré du mélange réactionnel, recyclé dans la zone d'addition de l'amine après la séparation de l'hydrogène chloré, est égale ou inférieure à 0,5 % du poids total du mélange réactionnel et le rapport molaire entre le phosgène et un groupe $NH_2$ des amines organiques est compris entre 12 : 1 et 200 : 1.

2. Procédé suivant la revendication 1, caractérisé en ce que la teneur en hydrogène chloré du mélange réactionnel est comprise entre 0,01 et 0,4 % du poids total de ce mélange.

3. Procédé suivant la revendication 1, caractérisé en ce que la réaction est réalisée dans un circuit mélangeur sous pression.

4. Procédé suivant la revendication 1, caractérisé en ce que le mélange réactionnel circule dans le circuit fermé par suite des différences dans les poids spécifiques.

5. Procédé suivant la revendication 1, caractérisé en ce que la température réactionnelle est réglée entre 100 et 220 °C.

6. Procédé suivant la revendication 1, caractérisé en ce que la pression est réglée entre 5 et 100 bars.

7. Procédé suivant la revendication 1, caractérisé en ce que 100 parties en poids du mélange réactionnel recyclé contiennent :

9,5 à 86 parties en poids de solvant organique,
4 à 40 parties en poids d'isocyanate organique,
10 à 50 parties en poids de phosgène et,
0,01 à 0,5 partie en poids d'hydrogène chloré.

8. Procédé suivant la revendication 1, caractérisé en ce que le rapport en volumes de la quantité totale du phosgène ajouté, du phosgène contenant du solvant ajouté et du mélange réactionnel circulant en circuit fermé à l'amine ou à la solution d'amine ajoutée est compris entre 300 : 1 et 1 : 1.

9. Procédé suivant la revendication 1, caractérisé en ce que les amines organiques sont choisies parmi les mono-, le di- et (ou) les poly-amines aliphatiques, cycloaliphatiques, aliphatiques-aromatiques et aromatiques.

10. Procédé suivant la revendication 1, caractérisé en ce que les amines organiques sont choisies parmi l'hexaméthylène-1,6-diamine, les mélanges d'hexaméthylène-1,6-diamine, de méthyl-2 pentaméthylène-1,5-diamine et d'éthyl-2 butylène-1,4-diamine, l'aminométhyl-3 triméthyl-3,5,5 cyclohexyl-amine, le diamino-2,4', le diamino-4,4' et le diamino-2,2' diphényl-méthane, les mélanges d'au moins deux de ces isomères, la toluylène-2,4- et la toluylène-2,6-diamine et leurs mélanges, les polyphényl-polyméthylène-polyamines et les mélanges de polyphényl-polyméthylène-polyamines et de diamino-diphényl-méthanes.

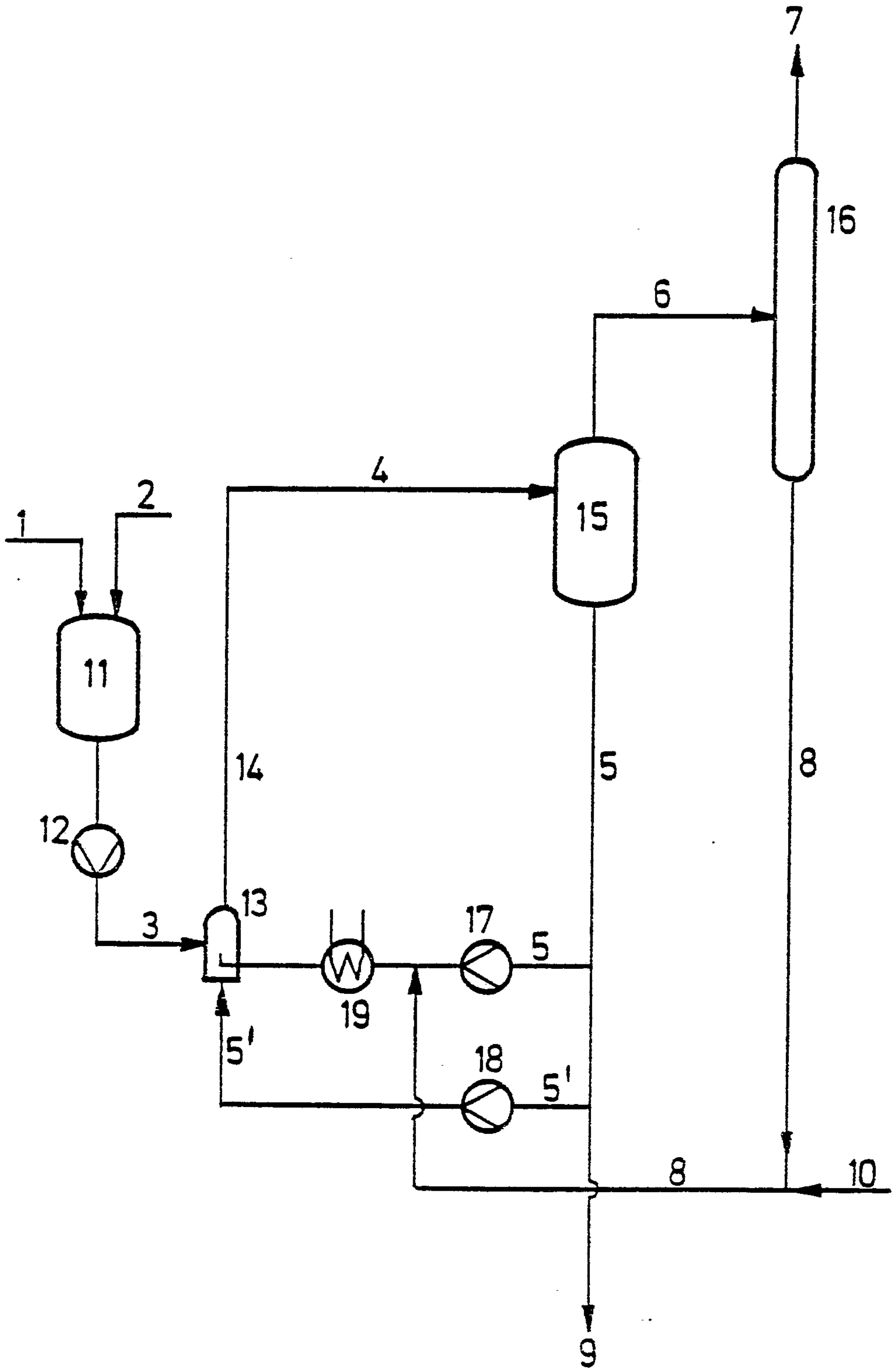

7
16
6
4
2
1
15
11
14
5
8
12
13
3
17
5
19
5'
18
5'
8
10
9